# EUROPEAN PATENT SPECIFICATION

(11) **EP 2 572 687 B1**
(45) Date of publication and mention of the grant of the patent: **20.04.2016**
(21) Application number: 11783587.6
(22) Date of filing: 18.05.2011
(51) Int. Cl.: A61F 13/49, A61F 13/15, A61F 13/53

(54) **BODILY ABSORBENT STRUCTURE AND METHOD FOR MANUFACTURING THE SAME**
ARTIKEL ZUR KÖRPERFLÜSSIGKEITSABSORPTION UND HERSTELLUNGSVERFAHREN DAFÜR
STRUCTURE ABSORBANTE DE FLUIDE CORPORELLE ET PROCEDE POUR PRODUIRE CELUI-CI

(30) Priority: 20.05.2010 JP 2010116782
(43) Date of publication of application: 27.03.2013
(73) Proprietor: Unicharm Corporation, Ehime 799-0111 (JP)
(72) Inventor: KAWAKAMI, Yusuke, Kanonji-shi Kagawa 769-1602 (JP); NAKAO, Hitomi, Kanonji-shi Kagawa 769-1602 (JP)
(74) Representative: Dolleymores
(86) International application number: PCT/JP2011/061443
(87) International publication number: WO 2011/145653

(56) References cited:
- EP-A1- 0 235 854
- EP-A1- 2 179 714
- EP-A1- 2 409 673
- EP-A1- 2 591 758
- EP-A1- 2 601 922
- JP-A- 2002 360 628
- JP-A- 2002 360 628
- JP-A- 2005 218 648
- JP-A- 2005 218 648
- JP-A- 2006 014 884
- JP-A- 2006 141 721
- US-A- 5 458 592

## Description

### {Technical Field}

The present invention relates to bodily fluid absorbent structures and methods for manufacturing the same and, more specifically, to bodily fluid absorbent structures which are capable of absorbing and containing a relatively large amount of bodily fluids and applicable to wearing articles such as urine absorbent pads, pantiliners, sanitary pads, sanitary napkins and disposable diapers, and to methods for manufacturing such bodily fluid absorbent structures.

### {Background}

Conventionally, bodily fluid absorbent structures including a liquid-absorbent core adapted to absorb and to contain bodily fluids and a core wrapping sheet formed of tissue paper adapted to wrap a liquid-absorbent core and to facilitate the diffusion of bodily fluids are known. For example, JP H8-196559 A (PTL 1) discloses a bodily fluid absorbent structure including a liquid-absorbent core including a mixture of superabsorbent polymer particles and fluff pulp, the fiber assembly layer lying on the upper surface of the core, and the core wrapping sheet adapted to wrap them.

### {Citation List}

### {Patent Literature}

{PTL 1}: JP H8-196559 A
JP 2005 218648 A relates to an absorbent article comprising a liquid-permeable surface sheet and a liquid-holding absorber. A groove is formed by compressing the surface sheet and the absorber. The surface sheet and the absorber are attached together by hot melt type adhesive to form a laminate.
JP 2002 360628 A discloses an absorptive article having a large number of almost circular recessed parts formed by integrally compressing a surface sheet and an absorbent on a surface sheet side surface, and has projecting parts formed by protruding a bottom surface of the recessed parts in the recessed parts.
EP2179714A1 discloses an absorbent article including: a base body that includes a fluid-permeable surface sheet, a fluid-impermeable back face sheet, and a liquid-absorbent core between the surface sheet and the back face sheet; and an absorbent body that includes a liquid-absorbent member. The absorbent body is placed on the fluid-permeable surface sheet of the base body.
EP 2 409 673 A1 is an A54 (3) EPC citation: it provides an absorbent article comprising a liquid-pervious surface sheet, a liquid-impervious leakage-preventive sheet, and a liquid-retentive absorption body disposed between the surface sheet and the leakage-preventive sheet. The absorption body comprises at least two layers, at least one of the layers is a first absorption body layer composed of a hydrophilic sheet and a super-absorbent polymer.

### {Summary}

### {Technical Problem}

In the bodily fluid absorbent structure disclosed in PTL 1, the liquid-absorbent core, the fiber assembly layer and the liquid-absorbent core wrapping sheet are bonded together with hot melt adhesives intermittently applied to the respective surfaces along which these constituents are put in contact with each other. This bodily fluid absorbent structure includes the fiber assembly layer lying on the upper surface of the liquid-absorbent core and, as a result, the bodily fluid absorbent structure is capable of absorbing and containing a relatively large amount of bodily fluids.

However, it is difficult to overlap the opposite lateral portions of the core wrapping sheet during the process of manufacturing due to the convex shape of the bodily fluid absorbent structure and, in consequence, the region in which the opposite lateral portions of the core wrapping sheet overlap each other might be partially curled up, and then the hot melt adhesives applied thereto might adhere to the manufacturing apparatus, thereby high-speed production might be interrupted.

To deal with such problems, it may be contemplated to secure the core wrapping sheet as a whole to the liquid-absorbent core by press working. However, in this case, there is a likelihood that the securing effect of the entirely compressed region might be released when the liquid-absorbent core absorbs bodily fluids and expands and eventually the core wrapping sheet might be separated from the liquid-absorbent core. If the compression area is enlarged and/or the pressure for the compression is increased to avoid the above-mentioned situation, the stiffness of the bodily fluid absorbent structure as a whole will be unacceptably increased and the desired flexibility will be deteriorated, and thereby deteriorate the feeling to wear of the article.

An object of the present invention is to provide a bodily fluid absorbent structure adapted to be stably secured in a state in which a liquid-absorbent core is wrapped with a core wrapping sheet and ensuring a high workability for a process of manufacturing the bodily fluid absorbent structure used for wearing articles, and a method for manufacturing the same.

### {Solution to Problem}

The present invention provides the bodily fluid absorbent structure of independent claim 1 and a method for manufacturing the same of claim 5. The dependent claims specify preferred by optional features.

A first aspect of this invention relates to a bodily fluid absorbent structure having a longitudinal axis and a transverse axis being orthogonal to the longitudinal axis, which includes a liquid-absorbent core containing at least water-absorbent fibers and a core wrapping sheet adapted to wrap the liquid-absorbent core entirely.

The first aspect of this invention further includes the following features:
the bodily fluid absorbent structure has first and second end regions spaced apart from and opposite to each other in a direction of the longitudinal axis and a middle region extending in the direction of the longitudinal direction between the first and second end regions;
on the side of a first surface, the core wrapping sheet and the liquid-absorbent core are bonded to each other by the intermediary of a bond region defined by a hot melt adhesive intermittently applied to substantially entire opposite surfaces of the core wrapping sheet and the liquid-absorbent core; and
on the side of a second surface, lateral portions of the core wrapping sheet are overlapped with each other, and the core wrapping sheet and the liquid-absorbent core are secured to each other in compressed end portions formed in the first and second end regions and a compressed middle portion formed in lateral portions of the middle region except a central zone as viewed in the direction of the transverse axis.

A second aspect of this invention relates to a method for manufacturing the bodily fluid absorbent structure, which method includes the steps of:
conveying a continuous web as material of the core wrapping sheet in a machine direction and coating the continuous web at given locations with adhesive;
placing the liquid-absorbent cores in respective region coated with the adhesive;
overlapping lateral portions of the continuous web extending in the machine direction each other and thereafter press working the continuous web to form the compressed end portions extending in a direction crossing the machine direction; and,
after the compressed end portions have been formed, press working the continuous web to form the compressed middle portions extending in the machine direction between the compressed end portions.

According to one embodiment of this invention, the mass of the water-absorbent fibers constituting the liquid-absorbent core is higher in the middle region than the remaining regions and, as a result, the liquid-absorbent core has a convex shape.

The compressed area ratio of a compression pattern defining the compressed end portions is in a range of 30 to 50% of the total area of the first and second end regions and the compressed area ratio of a compression pattern defining the compressed middle portions is in a range of 5 to 25% of the total area of the middle region.

According to even another embodiment of this invention, a liquid-pervious topsheet is laid on one of the side of the first surface and the second surface and hinge lines are formed so as to be convex from the topsheet toward the liquid-absorbent core.

According to still another embodiment of this invention, a range in which lateral portions of the core wrapping sheet are overlapped each other may be in one of lateral portions of the middle region.

### {Advantageous Effects of Invention}

In the bodily fluid absorbent structure according to one or more embodiments of this invention, on the side of the first surface, the core wrapping sheet is bonded to the liquid-absorbent core with a hot melt adhesive and, on the side of the second surface, the core wrapping sheet is secured to the liquid-absorbent core by the intermediary of the compressed end portions and the compressed middle portions. In this way, the core wrapping sheet may be stably secured to the liquid-absorbent core which is kept entirely wrapped by the core wrapping sheet and therefore the core wrapping sheet might be separated from the liquid-absorbent core. According to the manufacturing method according to this invention, the second surface side on which the lateral portions of the core wrapping sheet are overlapped with each other is not coated with adhesive and consequently the hot melt adhesive should not run off from the overlapping portions of the lateral portions and cling to part of the manufacturing apparatus.

### {Brief Description of Drawings}

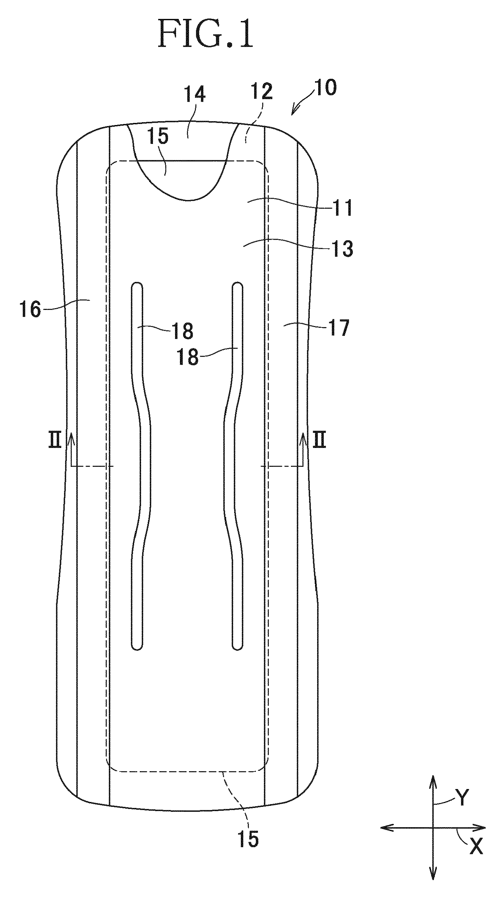

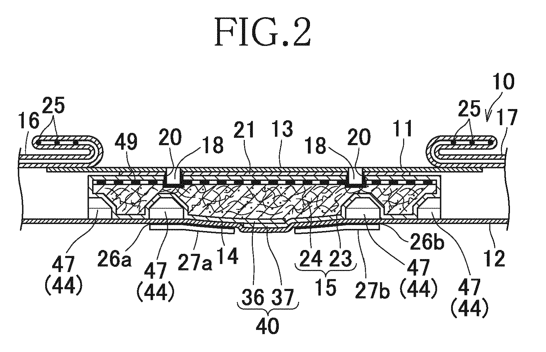

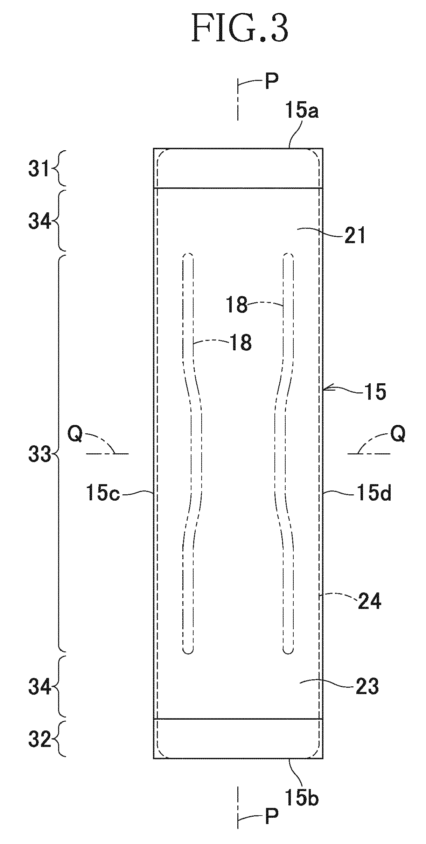

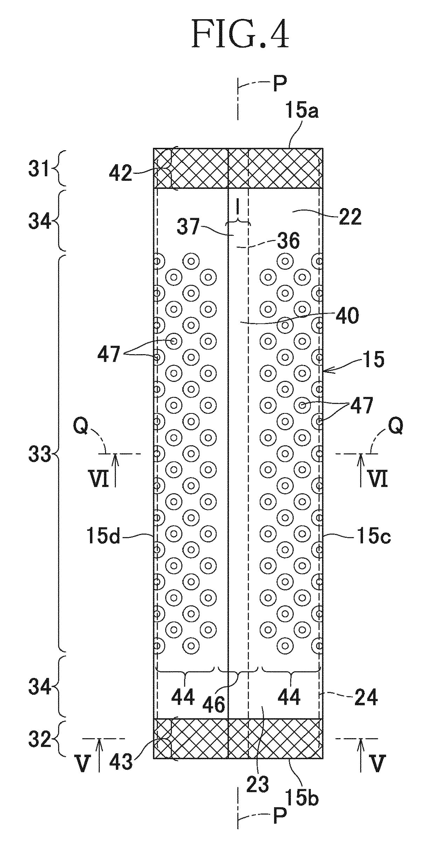

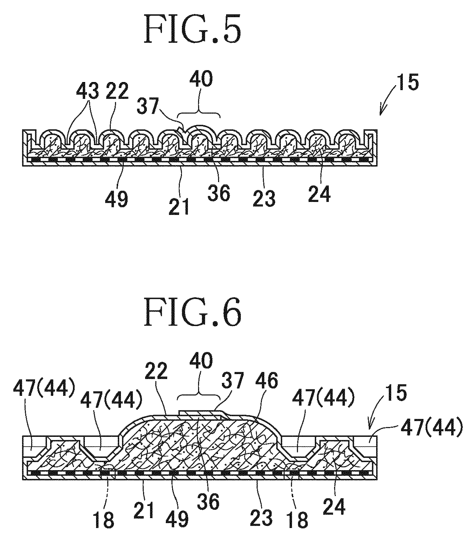

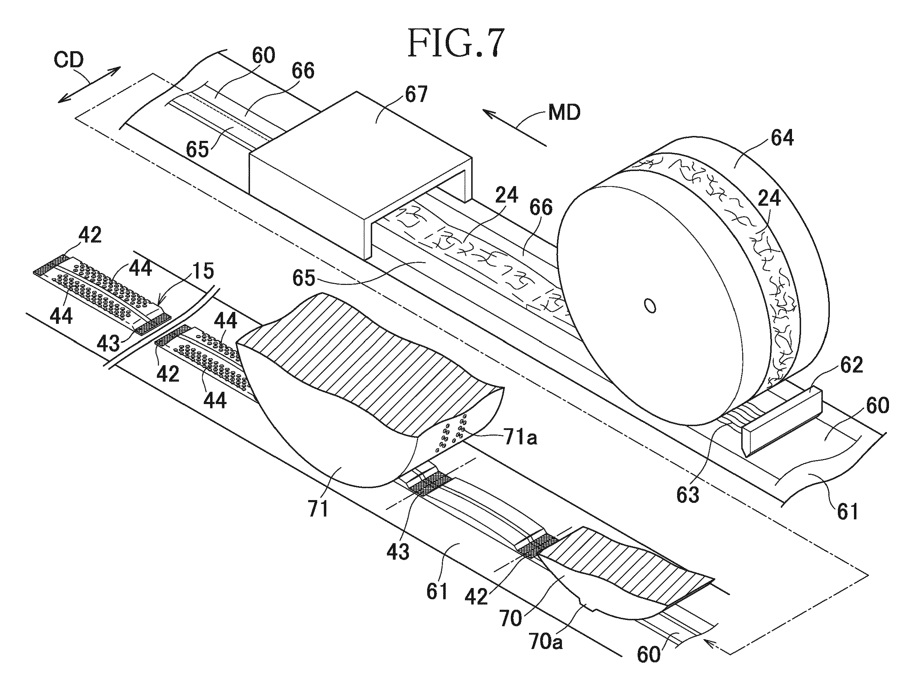

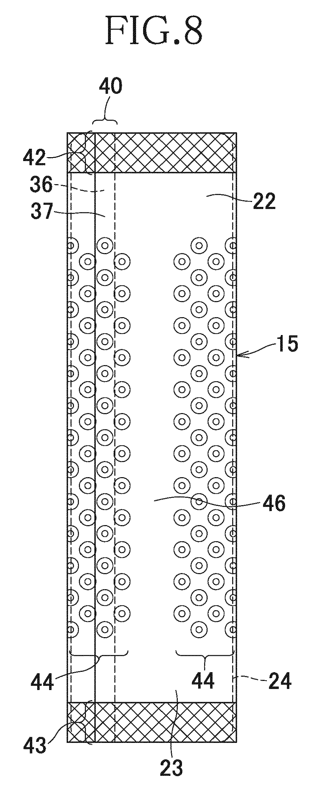

{Fig. 1} Fig. 1 is a plan view of a urine-absorbent pad as an example of a wearing article using a bodily fluid absorbent structure.
{Fig. 2} Fig. 2 is a sectional view taken along line II-II in Fig. 1.
{Fig. 3} Fig. 3 is a plan view of the bodily fluid absorbent structure as viewed from the side of a first surface thereof.
{Fig. 4} Fig. 4 is a plan view of the bodily fluid absorbent structure as viewed from the side of a second surface thereof.
{Fig. 5} Fig. 5 is a sectional view taken along line V-V in Fig. 4.
{Fig. 6} Fig. 6 is a sectional view taken along line VI-VI in Fig. 4.
{Fig. 7} Fig. 7 is a schematic diagram illustrating a process of manufacturing the bodily fluid absorbent structure.
{Fig. 8} Fig. 8 is a plan view similar to Fig. 4, illustrating another embodiment of the bodily fluid absorbent structure.

### {Description of Embodiments}

As specific embodiments of the wearing article using a bodily fluid absorbent structure 15, this invention will be described hereunder on the basis of a urine absorbent pad 10. Fig. 1 is a plan view of the urine absorbent pad 10, Fig. 2 is a sectional view taken along line II-II in Fig. 1, Fig. 3 is a plan view of the bodily fluid absorbent structure 15 as viewed from the side of a first surface 21 and Fig. 4 is a plan view of the bodily fluid absorbent structure 15 as viewed from the side of a second surface 22. In this regard, a hinge line 18 is indicated in Fig. 3 by an imaginary line for convenience of illustration.

Referring to Figs. 1 and 2, the urine absorbent pad 10 is shaped in a vertically long rectangle having a longitudinal direction Y and a transverse direction X and includes an upper surface 11, a lower surface 12, a liquid-pervious topsheet 13, a liquid-impervious backsheet 14, the bodily fluid absorbent structure 15 interposed between the topsheet 13 and the backsheet 14 and a pair of leakage barrier cuffs 16, 17 extending in the longitudinal direction Y along opposite lateral portions of the upper surface 11 of the topsheet 13. The upper surface 11 of the topsheet 13 is formed with a pair of hinge lines 18 in the form of grooves spaced apart from and opposed to each other in the transverse direction X and extending in the longitudinal direction Y into the bodily fluid absorbent structure 15. A cushion sheet 20 is interposed between the topsheet 13 and the bodily fluid absorbent structure 15.

The topsheet 13 may be formed, for example, of a liquid-pervious fibrous nonwoven fabric, a porous plastic film or a laminate sheet thereof and the backsheet 14 may be formed, for example, of a liquid-impervious but moisture-pervious plastic film, a liquid-impervious fibrous nonwoven fabric or a laminate sheet thereof. The cushion sheet 20 may be formed, for example, of a breathable and liquid-pervious fibrous nonwoven fabric to improve cushioning properties desired for the wearer's skin, to ensure bodily fluids are absorbed and contained at a central region of the urine absorbing pad 10 without leaving bodily fluids diffuse and, in addition, to separate the topsheet 13 and the bodily fluid absorbent structure 15 from each other and thereby to prevent bodily fluids from freely flowing back toward the topsheet 13. Though not illustrated, it is possible to use thermoplastic sheet members as material of the topsheet 13 and the cushion sheet 20 and to compress these sheet members under heating so that these sheet members may be integrally heat-sealed. According to the illustrated embodiments, respective sheet members overlapping each other are bonded to each other by known adhesive means such as hot melt adhesives.

Referring to Figs. 2 through 4, the bodily fluid absorbent structure 15 has a longitudinal axis P, a transverse axis Q being orthogonal to the longitudinal axis P, the first surface 21 and a second surface 22 opposite to the first surface 21 and is provided in the form of a substantially rectangular pad contoured by first and second ends 15a, 15b and opposite first and second side edges 15c, 15d extending in the direction of the longitudinal axis P between the first and second ends 15a, 15b. The bodily fluid absorbent structure 15 includes a liquid-pervious and liquid-diffusive core wrapping sheet 23 such as tissue paper and a liquid-absorbent core 24 entirely wrapped with the core wrapping sheet 23.

The liquid-absorbent core 24 is molded into a predetermined shape and has a rigidity higher than those of the sheet members such as the topsheet 13, the backsheet 14 and the core wrapping sheet 23. In other words, the liquid-absorbent core 24 is a semirigid panel formed from a mixture of superabsorbent polymer particles and water-absorbent fibers such as fluff pulp and optionally thermoplastic short fibers. While the mixture proportions of the superabsorbent polymer particles and the water-absorbent fibers may be appropriately varied depending on the absorption rate and the absorptive capacity required for the liquid-absorbent core 24, the mixture proportion of the superabsorbent polymer particles in the liquid-absorbent core 24 as a whole is preferably in a range of 35 to 65 %. This is because, if the mixture proportion thereof is less than 35%, a preventive function against rewetting phenomenon might be reduced and, if the mixture proportion exceeds 65%, the bodily fluid absorption rate might unacceptably decrease.

The core wrapping sheet 23 may be formed of tissue paper, a hydrophilic fibrous nonwoven fabric, a hydrophilized hydrophobic fibrous nonwoven fabric, a porous plastic film or a laminate sheet including these nonwoven fabrics and/or film. The core wrapping sheet 23 functions not only to improve diffusion of bodily fluids but also to ensure shape retention of the liquid-absorbent core 24 and, at the same time, to prevent the superabsorbent polymer particles from falling off. Furthermore, the core wrapping sheet 23 serves as carrier sheet in a process of manufacturing the bodily fluid absorbent structure 15 as will be described later.

As the superabsorbent polymer particles, it is possible to use water-insoluble polymeric hydrogels, for example, graft polymer of starch, modified cellulose or self-cross-linking type acrylic metal salts known as the absorbent material of absorbent articles such as sanitary napkins and disposable diapers, at a mass per unit area in a range of 100 to 600 g/m².

As an optional extra, thermoplastic branched staple fibers (split yarns or split fibers) of 15% or less by mass may be blended into the liquid-absorbent core 24 to ensure that these staple fibers intertwine with the fluff pulp fibers as well as the superabsorbent polymer particles to improve the shape retention of the liquid-absorbent core 24 and capillary action channels are formed between the staple fibers and the fluff pulp fibers to improve the bodily fluid-absorption capacity.

Referring again to Figs. 1 and 2, the leakage barrier cuffs 16, 17 respectively include sheet members formed of a liquid-impervious or poorly-liquid-pervious fibrous nonwoven fabric or a plastic film and a plurality of thread, strand or string elastic elements 25 arranged within sleeves defined by the respective sheet members having been folded back. The backsheet 14 is formed in a middle region with a pair of fastening regions 26a, 26b extending in the direction of the longitudinal axis P of the urine absorbent pad 10 and adapted to act on the wearer's garment wherein these fastening regions 26a, 26b are covered with separators 27a, 27b formed of, for example, a plastic film, a craft paper or a fibrous nonwoven fabric.

Referring to Fig. 3, the bodily fluid absorbent structure 15 will be sectioned hereunder for convenience of explanation into first and second end regions 31, 32, a middle region 33 lying in a middle in the direction of the longitudinal axis P and intermediate regions 34, lying between the first end section 31 and the middle region 33 and between the second end section 32 and the middle region 33, respectively.

The bodily fluid absorbent structure 15 has a convex shape in which a mass of the water-absorbent fibers constituting the liquid-absorbent core 24 is higher in the middle region 33 than in the remaining regions. More specifically, the mass of the water-absorbent fibers constituting the liquid-absorbent core 24 is in a range of 400 to 700 g/m² in the middle region 33 and in a range of 200 to 400 g/m² in the remaining regions, i.e., in the first and second end regions 31, 32 and the intermediate regions 34, respectively. The bodily fluid absorbent structure 15 having such a convex shape ensures high cushioning properties and makes it possible for the middle region 33 to absorb and contain relatively large amount of bodily fluids in a rapidmanner . In this regard, as will be described later in detail, the mass of the water-absorbent fibers in the first and second end regions 31, 32 may be set to be lower than that in the intermediate regions 33, for example, to a range of 100 to 300 g/m² in order that the first and second end regions 31, 32 may be subject to compression working in a correspondingly easy manner.

Referring to Fig. 4, lateral portions 36, 37 extending in the direction of the longitudinal axis P of the core wrapping sheet 22 and the vicinity thereof are folded onto the side of the second surface 22 and the both lateral portions 36, 37 are overlapped with each other in the vicinity of the longitudinal axis P of the bodily fluid absorbent structure 15 so that a region 40 in which the both side edges are overlapped with each other may extend in the direction of the longitudinal axis P. The overlapping portion 40 preferably has a dimension 1 in the direction of the transverse axis Q in a range of about 20.0 to 30.0 mm in order that the liquid-absorbent core 24 may be reliably wrapped.

The side of the second surface 22 of the bodily fluid absorbent structure 15 is subjected to pressurizing compression working in the first and second end regions 31, 32 and the lateral portions of the middle region 33 to form compressed end portions 42, 43 and compressed middle portions 44, respectively. In the compressed end portions 42, 43 as well as in the compressed middle portions 44, the core wrapping sheet 23 and the liquid-absorbent core 24 are secured to each other and, in these portions 42, 43, 44, the liquid-absorbent core 24 is in a sufficiently compressed state to ensure that the liquid-absorbent core 24 should not lose its initial shape even when the liquid-absorbent core 24 absorbs bodily fluids. The opposite end sections 42, 43 formed on the first and second end regions 31, 32, respectively, ensure to prevent part of the liquid-absorbent core 24, particularly the superabsorbent polymer particles from leaking out of the first and second end regions 31, 32 in the direction of the longitudinal axis P. In addition, since the compressed middle portions 44 are formed on both sides of the middle region 33 and not along a central zone 46 of the middle region 33 as viewed in the direction of the transverse axis Q, the central zone 46 of the middle region 33 is more bulky and correspondingly more flexible and cushiony than the remaining regions.

More specifically, the compressed endportions 42, 43 are formed in a lattice pattern at an area ratio in a range of 30 to 50% of the first and second end regions 31, 32, as a whole. The compressed middle portions 44 are formed at an area ratio in a range of 5 to 25% of the middle region 33 as a whole by the press working at a pressure higher than that at which the compressed end portions 42, 43 are formed and arranged in a zigzag compression pattern defined by a plurality of dots 47. While both the compressed end portions 42, 43 and the compressed middle portions 43 may be formed so as to have various shapes and sizes other than those as have been described above, when the compressed middle portions 44 are formed in the dot-like compression pattern as in the illustrated embodiment, the respective dots 47 preferably have a diameter in a range of about 0.5 to about 5.0 mm and a distance between each pair of the adjacent dots 47 is preferably in a range of about 2.0 to about 10.0 mm in order to prevent the compressed regions from being excessively hardened. The respective dots 47 are locally independent compressed regions which are tapered downward in a thickness direction of the liquid-absorbent core 24.

As illustrated in Fig. 4, the compressed middle portions 44 formed in the middle region 33 extend inward from the lateral portions 15c, 15d of the bodily fluid absorbent structure 15 and the dots 47 of the respective compressed middle portions 44 partially overlap the lateral portions 15c, 15d. For the reason that entire lateral portions of the middle region 33 inclusive of the lateral portions 15c, 15d are subj ected to the press working, the core wrapping sheet 23 and the liquid-absorbent core 24 are secured to each other in close contact with each other and therefore bodily fluids may be smoothly guided in the thickness direction of the bodily fluid absorbent structure 15.

Fig. 5 is a sectional view taken along line V-V in Fig. 4 and Fig. 6 is a sectional view taken along line VI-VI in Fig. 4.

Referring to Figs. 5 and 6, on the side of the first surface 21, the core wrapping sheet 23 and the liquid-absorbent core 24 are bonded to each other by the intermediary of a bond region 49 defined by a hot melt adhesive intermittently applied to the entire inner surface of the core wrapping sheet 23 with the amount of coating adjusted so as not to interfere with the desired liquid-absorption ability. On the side of the second surface 22, the core wrapping sheet 22 and the liquid-absorbent core 24 are secured to each other in the compressed end portions 42, 43 and the compressed middle portions 44 and, therefore, the core wrapping sheet 23 should not be separated from the liquid-absorbent core 24 and/or the liquid-absorbent core 24 should not lose its initial shape even if the urine pad 10 is bent or distorted during use of the urine absorbent pad 10.

On the side of the first surface 21, the core wrapping sheet 23 as a whole and the liquid-absorbent core 24 are almost evenly bonded to each other by the intermediary of the bond region 49, and thereby it is possible to inhibit a possible distortion of the bodily fluid absorbent structure 15, and also when it is desired to form the hinge lines 18 from above the topsheet 13, such hinge lines 18 may be relatively easily formed in a given shape.

While the first surface 21 of the bodily fluid absorbent structure 15 lies on the side of the upper surface 11 of the urine absorbent pad 10 and the second surface 22 lies on the side of the lower surface 12 of the urine absorbent pad 10 in this embodiment, an alternative arrangement is also possible in which the first surface 21 lies on the side of the lower surface 12 of the urine absorbent pad 10 and the second surface 22 lies on the side of the upper surface 11.

As the hot melt adhesive used to form the bond region 49, various types of hot melt adhesive widely used in the relevant technical field such as SIS-based hot melt adhesives, SBS-based hot melt adhesives and SEBS-based hot melt adhesives may be used without limitation and may be applied selectively in various patterns such as a solid pattern using a coater, a controlled seam pattern, a spiral pattern and a summit pattern. In this regard, when the first surface 21 lies on the side of the upper surface 11 of the urine absorbent pad 10, the hot melt adhesive is preferably applied in the intermittent pattern such as a spiral pattern in consideration of the bodily fluid absorbent performance as well as the bond strength. When the first surface 21 lies on the side of the lower surface 12, the hot melt adhesive is preferably applied in the solid pattern using a coater in order to ensure a desired bond strength.

Fig. 7 is a schematic diagram illustrating by way of example a process of manufacturing the bodily-fluid absorbent structure 15. In Fig. 7, a machine direction is designated by MD and a cross direction being orthogonal to the machine direction MD is designated by CD.

Referring to Fig. 7, the process of manufacturing the bodily fluid absorbent structure 15 starts with conveyance of a continuous web 60 loaded on a conveyor belt 61 is conveyed in the machine direction MD and, in an adhesive coating station 62, coated on its given area (corresponding to the entire area of the first inner surface 21) with a hot melt adhesive continuously in the machine direction MD. Then, at a core supplying station 64 defined by a rotary drum provided with a suction mechanism, the liquid-absorbent cores 24 shaped into the pads are successively transferred onto adhesive coated regions 63 of the continuous web 60. After the liquid-absorbent cores 24 have been transferred onto the adhesive coated regions 63, lateral portions 65, 66 (corresponding to the lateral portions 36, 37) of the continuous web 60 extending in the machine direction MD are folded inward and overlapped with each other by a folding guide 67.

The continuous web 60 of which the lateral portions 65, 66 are kept in an overlapped state is conveyed in the machine direction MD to a first press working station 70 defined by a rotary roll formed on its peripheral surface with a plurality of protrusions 70a, at which given regions of the continuous web 60 are press worked to form the compressed end portions 42, 43 extending in the cross direction CD. The respective end sections' compressed regions 42, 43 are spaced apart from each other in the machine direction MD by a given dimension and formation of these compressed end portions 42, 43 ensures to prevent the lateral portions 65, 66 of the continuous web 60 from being distorted in the course of being conveyed and, at the same time, to prevent each of the liquid-absorbent cores 24 having been transferred onto given sections (corresponding to the bodily fluid absorbent structures 15) from partially moving to the adjacent sections. Then, the continuous web 60 enters a second press working station 71 defined by a rotary roll formed on its peripheral surface with a plurality of dot-like deboss patterns and, in this second press working station 71, the continuous web 60 is press worked on both sides of the overlapping lateral portions 65, 66 to form the compressed middle portions 44. After the compressed middle portions 44 have been formed, the continuous web 60 is cut at given regions to form the individual bodily fluid absorbent structures 15.

In this process, on the side of the continuous web 60 on which the lateral portions 65, 66 are overlapped with each other, the continuous web 60 and the liquid-absorbent cores 24 are secured to each other not with hot melt adhesive but by the intermediary of the compressed end portions 42 and the compressed middle portions 44. Consequently, hot melt adhesive should not run off from the overlapping portions of the lateral portions 65, 66 and cling to a part of the manufacturing apparatus.

Fig. 8 is a plan view similar to Fig. 4, illustrating another embodiment of the bodily fluid absorbent structure according to this invention. The basic construction of the bodily fluid absorbent structure 15 according to this embodiment is similar to that of the first embodiment and therefore the description of this embodiment given hereunder will be limited to one or more features distinguished from those of the first embodiment.

According to this embodiment, on the side of the second surface 22 of the bodily fluid absorbent structure 15, the lateral portions 36, 37 of the core wrapping sheet 23 overlap each other not in the central zone 46 but in one lateral region in the direction of the transverse axis Q of the bodily fluid absorbent structure 15 and one of the compressed middle portions 44 is formed in the overlapping portion 40 of the lateral portions 36, 37. The compressed middle portions 44 formed in the overlapping portion 40 ensures it to prevent the superabsorbent polymer particles from falling off.

### {Reference Signs List}

13 topsheet
15 bodily fluid absorbent structure
18 hinge line
21 first surface
22 second surface
23 core wrapping sheet
24 liquid-absorbent core
31 first end section
32 second end section
33 middle region
36, 37 lateral portions of core wrapping sheet
40 overlapping portion of core wrapping sheet's lateral
portions
42, 43 compressed end portions
44 compressed middle portions
46 central zone of midsection
49 adhesive region
60 continuous web
65, 66 lateral portions of continuous web
MD machine direction
CD cross direction
P longitudinal axis
Q transverse axis

## Claims

1. A bodily fluid absorbent structure (15) having a longitudinal axis (P) and a transverse axis (Q) being orthogonal to the longitudinal axis, comprising:
a liquid-absorbent core (24) containing at least water-absorbent fibers; and
a core wrapping sheet (23) adapted to wrap the liquid-absorbent core (24) entirely, wherein:
the bodily fluid absorbent structure (15) has first and second end regions (31, 32) spaced apart from and opposite to each other in the direction of the longitudinal axis and a middle region (33) extending in the direction of the longitudinal direction between the first and second end regions (31,32);
on the side of a first surface (21), the core wrapping sheet (23) and the liquid-absorbent core (24) are bonded to each other by the intermediary of a bond region (49) defined by hot melt adhesive intermittently appliedto substantially entire opposite surfaces of the core wrapping sheet (23) and the liquid-absorbent core (24);
on the side of a second surface (22), lateral portions (36, 37) of the core wrapping sheet (23) are overlapped with each other, and the core wrapping sheet (23) and the liquid-absorbent core (24) are secured to each other in compressed end portions (42, 43) formed in the first and second end regions (31,32) and compressed middle portions (44) formed in lateral portions of the middle region (33) except a central zone (46) as viewed in the direction of the transverse axis, and
a compressed area ratio of a compression pattern defining the compressed end portions is in a range of 30 to 50% of a total area of the first and second end regions and a compressed area ratio of a compression pattern defining the compressed middle portions is in a range of 5 to 25% of the total area of the middle region.

2. The bodily fluid absorbent structure (15) according to claim 1, wherein the mass of the water-absorbent fibers constituting the liquid-absorbent core (24) is higher in the middle region than the remaining regions and, as a result, the liquid-absorbent core (24) has a convex shape.

3. The bodily fluid absorbent structure (15) according to claim 1 or 2, wherein a liquid-pervious topsheet (13) is laid on one of the side of the first surface (21) and the second surface (22) and hinge lines (18) are formed so as to be convex from the topsheet (13) toward the liquid-absorbent core (24).

4. The bodily fluid absorbent structure (15) according to any one of claims 1 through 3, wherein lateral portions (36,37) of the core wrapping sheet (23) are overlapped with each other may be in one of lateral portions of the middle region (33).

5. A method for manufacturing a bodily fluid absorbent structure (15) according to any one of claims 1 through 4, the method including the steps of:
conveying a continuous web (60) as material of the core wrapping sheet (23) in a machine direction (MD) and coating the continuous web (60) at given locations with adhesive;
placing the liquid-absorbent cores (24) in respective regions coated with the adhesive;
overlapping lateral portions (65, 66) of the continuous web (60) extending in the machine direction with each other and thereafter press working the continuous web (60) to form the compressed endportions (42,43) extending in a direction crossing the machine direction; and,
after the compressed end portions (42,43) have been formed, press working the continuous web (60) to form the compressed middle portions (44) extending in the machine direction between the compressed end portions (42,43).

## Patentansprüche

1. Körperflüssigkeit absorbierende Struktur (15) mit einer Längsachse (P) und einer senkrecht zur Längsachse verlaufenden Querachse (Q), umfassend:
einen Flüssigkeit absorbierenden Kern (24), der mindestens Wasser absorbierende Fasern enthält; und
eine Kernhüllfolie (23), die zum vollständigen Umhüllen des Flüssigkeit absorbierenden Kerns (24) eingerichtet ist, wobei:
die Körperflüssigkeit absorbierende Struktur (15) erste und zweite Endbereiche (31, 32), die voneinander beabstandet sind und einander in der Richtung der Längsachse gegenüber liegen, und einen mittleren Bereich (33), der sich in der Längsrichtung zwischen den ersten und zweiten Endbereichen (31, 32) erstreckt, hat;
auf der Seite einer ersten Oberfläche (21) die Kernhüllfolie (23) und der Flüssigkeit absorbierende Kern (24) unter Zwischenschaltung eines Verbindungsbereichs (49), der von intermittierend auf im Wesentlichen gesamte gegenüber liegende Oberflächen der Kernhüllfolie (23) und des Flüssigkeit absorbierenden Kerns (24) aufgetragenen Schmelzklebstoff definiert ist, miteinander verbunden sind;
auf der Seite einer zweiten Oberfläche (22) Seitenpartien (36, 37) der Kernhüllfolie (23) einander überlappen und die Kernhüllfolie (23) und der Flüssigkeit absorbierende Kern (24) in komprimierten Endpartien (42, 43), die in den ersten und zweiten Endbereichen (31, 32) ausgebildet sind, und in komprimierten Mittelpartien (44), die in Seitenpartien des Mittelbereichs (33) mit Ausnahme einer in Richtung der Querachse betrachteten Mittelzone (46) ausgebildet sind, aneinander befestigt sind, und
ein Verhältnis der komprimierten Flächen eines die komprimierten Endpartien definierenden Kompressionsmusters im Bereich von 30 bis 50% einer Gesamtfläche der ersten und zweiten Endbereiche liegt und ein Verhältnis der komprimierten Flächen eines die komprimierten Mittelpartien definierenden Kompressionsmusters im Bereich von 5 bis 25% der Gesamtfläche des Mittelbereichs liegt.

2. Körperflüssigkeit absorbierende Struktur (15) nach Anspruch 1, wobei die Masse der den Flüssigkeit absorbierenden Kern (24) bildenden Wasser absorbierenden Fasern im Mittelbereich größer ist als in den übrigen Bereichen und der Flüssigkeit absorbierende Kern (24) deshalb eine Konvexform hat.

3. Körperflüssigkeit absorbierende Struktur (15) nach Anspruch 1 oder 2, wobei eine flüssigkeitsdurchlässige Oberlage (13) auf die Seite der ersten Oberfläche (21) oder der zweiten Oberfläche (22) gelegt ist und Scharnierlinien (18) so ausgebildet sind, dass sie von der Oberlage (13) aus in Richtung des Flüssigkeit absorbierenden Kerns (24) konvex sind.

4. Körperflüssigkeit absorbierende Struktur (15) nach einem der Ansprüche 1 bis 3, wobei Seitenpartien (36, 37) der Kernhüllfolie (23) einander möglicherweise in einer Seitenpartie des Mittelbereichs (33) überlappen.

5. Verfahren zur Herstellung einer Körperflüssigkeit absorbierenden Struktur (15) nach einem der Ansprüche 1 bis 4, umfassend die folgenden Schritte:
das Fördern einer Endlosbahn (60) als Material der Kernhüllfolie (23) in einer Maschinenrichtung (MD) und das Beschichten der Endlosbahn (60) mit Klebstoff an gegebenen Stellen;
das Auflegen der Flüssigkeit absorbierenden Kerne (24) auf jeweils mit dem Klebstoff beschichtete Bereiche;
das Überlappen von Seitenpartien (65, 66) der in der Maschinenrichtung verlaufenden Endlosbahn (60) mit einander und danach das Pressen der Endlosbahn (60) zur Bildung der sich in einer quer zur Maschinenrichtung verlaufenden Richtung erstreckenden komprimierten Endpartien (42, 43); und
nach der Bildung der komprimierten Endpartien (42, 43) das Pressen der Endlosbahn (60) zur Bildung der sich in zwischen den komprimierten Endpartien (42, 43) in der Maschinenrichtung erstreckenden komprimierten Mittelpartien (44).

## Revendications

1. Structure (15) absorbant les fluides corporels qui a un axe longitudinal (P) et un axe transversal (Q) qui est perpendiculaire par rapport à l'axe longitudinal, comportant :
une partie centrale (24) absorbant les liquides qui contient au moins des fibres absorbant l'eau ; et
une feuille formant enveloppe (23) pour partie centrale qui est adaptée pour envelopper la partie centrale (24) absorbant les liquides dans son intégralité, dans laquelle :
la structure (15) absorbant les fluides corporels a des première et deuxième régions d'extrémité (31, 32) espacées et à l'opposé l'une par rapport à l'autre dans la direction de l'axe longitudinal et une région médiane (33) s'étendant dans la direction de l'axe longitudinal entre les première et deuxième régions d'extrémité (31, 32) ;
du côté d'une première surface (21), la feuille formant enveloppe (23) pour partie centrale et la partie centrale (24) absorbant les liquides sont liées l'une par rapport à l'autre par l'intermédiaire d'une région de liaison (49) définie par un adhésif thermofusible appliqué sur sensiblement l'intégralité des surfaces opposées de la feuille formant enveloppe (23) pour partie centrale et la partie centrale (24) absorbant les liquides ;
du côté d'une deuxième surface (22), des parties latérales (36, 37) de la feuille formant enveloppe (23) pour partie centrale se chevauchent l'une par rapport à l'autre, et la feuille formant enveloppe (23) pour partie centrale et la partie centrale (24) absorbant les liquides sont assujetties l'une par rapport à l'autre dans des parties comprimées (42, 43) formées dans les première et deuxième régions d'extrémité (31, 32) et des parties médianes comprimées (44) formées dans des parties latérales de la région médiane (33) à l'exception d'une zone (46) comme étant vue dans la direction de l'axe transversal, et
un rapport de surface comprimée d'un motif de compression définissant les parties d'extrémité comprimées se trouve dans une plage allant de 30 à 50 % d'une surface totale des première et deuxième régions d'extrémité et un rapport de surface comprimée d'un motif de compression définissant les parties médianes comprimées se trouve dans une plage allant de 5 à 25 % de la surface totale de la région médiane.

2. Structure (15) absorbant les fluides corporels selon la revendication 1, dans laquelle la masse des fibres absorbant l'eau constituant la partie centrale (24) absorbant les liquides est supérieure dans la région médiane que dans les autres régions, et, en conséquence, la partie centrale (24) absorbant les liquides a une forme convexe.

3. Structure (15) absorbant les fluides corporels selon la revendication 1 ou la revendication 2, dans laquelle une feuille de dessus (13) perméable aux liquides est posée sur l'une parmi la première surface (21) et la deuxième surface (22) et des lignes de charnière (18) sont formées de manière à être convexe depuis la feuille de dessus (13) jusqu'à la partie centrale (24) absorbant les liquides.

4. Structure (15) absorbant les fluides corporels selon l'une quelconque des revendications 1 à 3, dans laquelle des parties latérales (36, 37) de la feuille formant enveloppe (23) pour partie centrale se chevauchent l'une par rapport à l'autre et peuvent être dans l'une des parties latérales de la région médiane (33).

5. Procédé de fabrication d'une structure (15) absorbant les fluides corporels selon l'une quelconque des revendications 1 à 4, le procédé comprenant les étapes consistant à :
transporter une bande continue (60) pour le matériau de la feuille formant enveloppe (23) pour partie centrale dans une direction allant dans le sens machine (MD) et enduire la bande continue (60) au niveau d'emplacements donnés au moyen d'adhésif ;
placer les parties centrales (24) absorbant les liquides dans des régions respectives enduites au moyen d'adhésif ;
faire chevaucher des parties latérales (65, 66) de la bande continue (60) s'étendant dans la direction machine l'une par rapport à l'autre et par la suite faire travailler à la presse la bande continue (60) afin de former les parties d'extrémité comprimées (42, 43) s'étendant dans une direction croisant la direction allant dans le sens machine ; et,
une fois que les parties d'extrémité comprimées (42, 43) ont été formées, faire travailler à la presse la bande continue (60) afin de former les parties médianes comprimées (44) s'étendant dans la direction allant dans le sens machine entre les parties d'extrémité comprimées (42, 43).
